# EUROPEAN PATENT APPLICATION

(11) **EP 4 265 707 A1**
(43) Date of publication of application: **25.10.2023**
(21) Application number: 23167840.0
(22) Date of filing: 13.04.2023
(51) Int. Cl.: C11B 1/02, C11B 1/04, C11B 1/10, C11C 3/00

(54) **A METHOD FOR FATTY ACID ALKYL ESTER SYNTHESIS AND THEIR EXTRACTION FROM OLEAGINOUS MICROBES**

(30) Priority: 21.04.2022 IN 202221023478
(71) Applicant: Indian Oil Corporation Limited, 400051 Maharashtra Mumbai (IN); Department of Biotechnology, Ministry of Science and Technology, Government of India, New Delhi 110003 (IN)
(72) Inventor: SINGH, Dilip, Maharashtra (IN); SHARMA, Ajay Kumar, Maharashtra (IN); MATHUR, Anshu Shankar, Maharashtra (IN); GUPTA, Ravi Prakash, Maharashtra (IN)
(74) Representative: Pons

(57) **Abstract**

The present invention relates to a method for *in-situ* synthesis of alkyl esters of fatty acids and their efficient extraction from oleaginous microbes. Particularly, the present invention provides a rapid method for downstream processing of oleaginous biomass for *in-situ* synthesis of alkyl esters of fatty acids and their efficient extraction. The method of the present invention results in increased transesterification efficiency (>95%) of FAAE production from wet biomass, while eliminating the necessity of biomass harvesting, drying and large requirement of chemical solvents.

## Description

### FIELD OF THE INVENTION

The present invention relates to a method for *in-situ* synthesis of alkyl esters of fatty acids and their efficient extraction from oleaginous microbes. Particularly, the present invention provides a rapid method for downstream processing of oleaginous biomass for *in-situ* synthesis of alkyl esters of fatty acids and their efficient extraction. The method of the present invention results in increased transesterification efficiency (>95%) from wet biomass, while eliminating the necessity of biomass harvesting, drying and large requirement of chemical solvents.

### BACKGROUND OF THE INVENTION

Oil derived from different oleaginous feedstocks have the potential to meet or mitigate the rising demand for crude oil-derived diesel fuel, since long term supply of conventional fossil fuel is unsustainable due to rapid depletion of fossil fuel reserves. Being a renewable energy source, biodiesel has multiple advantages over crude oil derived diesel in terms of CO₂ emission reduction, sustainability as well as economic and social benefits to the local community. Oil from these feedstocks is converted into biodiesel by transesterification reaction, which can be then used directly or in mixture with diesel fuel. Biodiesel is classified into three generations i.e., first generation, second generation, and third generation biodiesel based on the feedstock source. First generation biodiesel is produced from edible oils such e.g., soybean oil, canola oil, palm oil etc., however long-term commercial viability is questionable due to its conflict with food supply, since significant amount of oil needs to be diverted for biodiesel production to meet commercial demand. Second generation biodiesel, produced from non-edible crops such as e.g., Karanjia oil, jatropha etc. was hailed as potential replacement however, these crops suffer from low oil productivity, requirement of higher investment on water, fertilizers and large-scale land for cultivation thus making them unsuitable for commercial scale biodiesel production.

Oleaginous microbes such as microalgae, yeasts have several advantages over first and second-generation feedstocks due to their high growth rate and oil productivity. These microbes can accumulate oil from 20% to 80% of dry biomass within 2-4 days of fermentation thus, exceeding the oil productivity by almost thousand times over superior oil crops such as palm, soybean, coconut etc. Since these microbes have very high oil productivity rate thus land requirement for commercial biodiesel production is far lower than oil crops (Chisti et al., 2007 Biotechnology Advances). All these properties make oleaginous microbial biomass a suitable feedstock for biodiesel production.

Although microbial feedstocks offer several advantages over bioenergy crops, still commercial success of third generation biodiesel will depend on its cost competitiveness against crude oil derived diesel. Oleaginous microbes need to have very high growth rate and oil productivity while utilizing low-cost nutrients for cultivation. Along with this, downstream processing of oleaginous biomass needs to be cost and energy efficient to produce biodiesel. Several reports have indicated that overall production cost of biodiesel can be significantly reduced if oleaginous microbes are producing high value co-products along with lipids for biodiesel. These co-products can be vitamins, antioxidants, pigments, carotenoids, omega-3 fatty acids etc.

Further, concurrent production of biodiesel along with the omega-3 fatty acids will be helpful to bring down the cost and will help to compete against crude oil diesel. Therefore, the selection of right microbial strain, producing lipids and omega-3 fatty acids, along with efficient downstream processing method is one of the key factors for commercialization of third generation biodiesel production. Downstream method for biodiesel production and omega-3 fatty acid extraction from microbial biomass needs to be carefully developed and optimized because omega-3 fatty acids are highly unstable and very prone to oxidation or epoxidation due to presence of multiple double bonds. Moreover, the conventional method of downstream processing of microalgal or yeast or bacterial biomass involves dewatering of fermentation broth by means of centrifugation, flocculation (auto or induced), floatation, filtration, and sedimentation etc. This biomass paste still contains more than 80% of water therefore, this is then dried with drum dryer, spray dryer, vacuum oven dryer, hot air dryer, freeze dryer etc. to remove remaining water. Drying of biomass is necessary before oil extraction since water molecules in the cell form hydrophilic layer around the cell and lipid body membrane thus, preventing solvents to access and solubilize the lipid efficiently. This results into incomplete lipid extraction hence, affecting overall lipid extraction yield. Harvesting and drying are one of the major energy intensive steps in microbial lipid production system, accounting for over 50%-70% of the total energy consumption in obtaining biodiesel from microbial biomass. This disturbs overall cost and energy economics of third generation biodiesel. Therefore, biodiesel from microalgae is still economically unviable despite significant progress in increasing microalgal biomass and lipid productivity rates, partly due to higher dewatering cost associated with harvesting and drying. Mata *et al*., 2010; Verma *et al*., 2010, Grima *et al*., 2003 in their papers reported harvesting cost of 20% to 30% of total microalgae production cost, while applying different harvesting techniques such as centrifugation, flocculation, floatation, filtration and sedimentation etc. Singh and Patidar *et al*., 2018 reviewed different harvesting techniques and concluded that biomass harvesting and drying can cost up to 90% of the total biomass production cost, while increasing overall energy input in the process. Sander and Murthy *et al*., 2010 revealed drying of biomass paste consumes more than 60% of the overall energy input thus resulting in a negative energy balance for producing algal biodiesel.

Efforts have been made to develop cost and energy efficient harvesting and drying methods. Innovative materials such as hydrogels have been tried in harvesting of microalgae by absorbing water from slurry.

US20170088811A1 describes once such use of hydrogels where stimuli sensitive hydrogels were used for biomass harvesting. Different stimuli such as pH and temperature were used for activation of gel. Although this method showed significant improvement in water removal from biomass slurry however, higher cost associated with hydrogel formation along with the large harvesting time makes it commercially unsuitable for deploying in the field.

CN2013102449307A describes innovative use of magnetic material in harvesting microalgae. Magnetic materials capable of adsorbing microalgae are added in medium and then magnetic field is applied to separate microalgal biomass. The process was very quick, scalable, and nontoxic to harvest the cells, however cost of such magnetic nanoparticle synthesis is still high.

US20130102055A1 mentions a continuous process of flocculation and deflocculation for microalgal biomass harvesting. Process involves continuous addition of flocculating salts in algae broth followed by harvesting and then recycling back the clean liquid for the cultivation of microalgae. This process provides significant cost benefits not only in terms of nutrient recycling but also in terms of process productivity since a continuous process is inherently fast over batch or semi continuous process.

US2012O129244A1 describes a method and systems where electromagnetic field is used for harvesting of microalgae biomass. This biomass was then dried and used to extract useful lipids from that while de-oiled biomass was used for nutrient and CO₂ recovery along with biogas production. Further, in the document, clean water was used to recycle back the nutrients in microalgae cultivation.

Combination of different harvesting methods such as centrifugation, flocculation, floatation, filtration, and sedimentation etc. were used in patent application numbers WO2015121618A1, CN102696340A, WO2013055887A1. All these combinations were tried to increase the harvesting efficiency. Concurrent harvesting and drying methods have been developed to reduce the energy input of the process.

CN203582856U discloses drawing of a utility model for large-scale microalgae separation, collection and drying. Biomass is continuously hopped from the harvesting tank and exposed to hot air blast for drying.

Further, in conventional approach, harvested biomass is subjected to drying with drum dryer, spray dryer, vacuum oven dryer, hot air dryer, freeze dryer etc. before proceeding for lipid extraction and transesterification for FAME or FAEE synthesis. As reported earlier, this whole process can cost up to 90% of the total biomass production cost while consuming more than 60% of the total input energy. There have been attempts to reduce cost and energy footprint of the process by replacing conventional extraction transesterification process with direct transesterification of dry or wet biomass.

US009416336B2 discloses about direct transesterification of algal biomass for FAEE production using acid catalyzed reaction. In the document, a process was developed using very low amount of chemicals solvents as compared to existing processes however when this method was applied on wet biomass by inventors, FAEE yield drastically reduced even with co-solvent.

Johnson *et al*., in 2009 reported similar trend of low biodiesel yield when wet biomass of *Thraustochytrids* was subjected to direct transesterification using acid catalysts in co-solvent systems. This underlines the importance of complete water removal from biomass for direct transesterification as reported earlier by Laurens *et al*., 2020, Bart *et al*., 2010, Yoo *et al*., 2015. Large amount of solvent is added in the reaction mixture to overcome the negative effect of water on transesterification reaction. Harvey *et. al.* 2011 used biomass to solvent ration of 1:600 for alkali catalyzed direct transesterification reaction.

US20150252285A1 describes the process for lipid extraction from wet biomass using co-solvent system along with pressurized CO₂. Although inventors mentioned scalability of the process, but lipid extraction yield is significantly down, when moisture content of the slurry exceeded above 40%.

Hechun Cao *et al*., 2013 investigates the optimal conditions required for converting wet microalgae biomass into biodiesel through *in situ* transesterification. The document reported that under the optimal conditions of 100mg dry weight equivalent wet microalgae biomass, 4mL methanol, 8mL n-hexane, 0.5M H₂SO₄, 120_{°}C, and 180 minutes reaction time, the biodiesel yield reached as high as 92.5% and the FAME content was 93.2%. However, the document indicates that biodiesel yield decreased from 91.4% to 10.3% as water content increased from 0% to 90% and emphasized that higher temperature could compensate the negative effect.

P.D. Patil *et al*., 2013 describes a single-step microwave-mediated supercritical ethanol extraction and transesterification process for algal (*N. salina*) biomass. Further, the process is based on the use of a passive heating element made of silicon carbide (SiC) to augment the microwave-mediated heating process at high temperatures and which was found to improve algae extraction efficiency, reduces extractive-transesterification time, and increases biodiesel yield.

US 9,416,336B2 describes a method of converting lipids in a lipid containing biological material into fatty acid ethyl esters (FAEE) wherein the method involves mixing biological material comprising lipids and biomass with a first non-polar solvent at a biomass: first nonpolar solvent ratio of 1:1 to 1:10 to form a first reaction mixture; mixing the first reaction mixture with ethanol and a liquid acid catalyst to generate a second reaction mixture at a biomass: catalyst ratio of 1:0.1 to 1:2 and a biomass: ethanol ratio of 1:1 to 1:10; and heating the second reaction mixture to a temperature of 50-75°C for a period of 4-8 hours to generate an ester mixture comprising at least some of the lipids converted into an FAEE product.

Ji-Yeon Park *et al*., 2014 provides different approaches used for direct transesterification by using enzymes, homogeneous and heterogeneous catalysts, ultrasound, microwave, and supercritical process.

E.A. Ehimen *et al*., 2009 provides an overview which highlights the effect of different reaction variables on the production of biodiesel from nonedible microalgae lipids, using the acid-catalyzed *in situ* transesterification process.

Although several reports and studies are available, however there is a need in the art to develop a rapid and less energy intensive downstream processing method for alkyl ester synthesis and their extraction directly from wet biomass slurry, without requiring biomass harvesting, drying, large number of chemical solvents or complete removal of moisture.

### SUMMARY OF THE INVENTION

In an aspect of the present invention, there is provided a rapid downstream processing method for synthesis and extraction of fatty acid alkyl ester from an oleaginous microbe, the method comprising:
(i) subjecting wet biomass slurry to osmotic and acidic treatment;
(ii) mixing the treated biomass slurry of step (i) having moisture content in the range of 80% - 90% with an extraction mixture;
(iii) adding non-polar solvent to the mixture of step (ii) to obtain an extract;
(iv) subjecting the extract to evaporation and treating with a polar solvent; and
(v) adding moisture adsorbent to the extract to obtain fatty acid alkyl ester (FAAE) rich in omega-3 fatty acids with the non-polar solvent,
wherein the addition of moisture adsorbent in step (v) increases the FAEE transesterification efficiency up to 95%.

In an embodiment of the present invention, there is provided a method, wherein the obtained fatty acid alkyl ester is selected from methyl ester, ethyl ester and propyl ester.

In another embodiment of the present invention, there is provided a method, wherein in step (ii) the treated biomass slurry is mixed with extraction mixture and is agitated at 100 rpm at a temperature of 90°C for a duration 1 hour.

In yet another embodiment of the present invention, there is provided a method, wherein the oleaginous microbe is a microalga.

In still another embodiment of the present invention, there is provided a method, wherein the wet biomass slurry is directly harvested from the reactor.

In an embodiment of the present invention, there is provided a method, wherein the osmotic treatment in step (i) comprises treating the wet biomass slurry with single distilled water or double distilled water or triple distilled water or water having very low TDS.

In another embodiment of the present invention, there is provided a method, wherein acidic treatment in step (i) comprises treating the wet biomass slurry with an organic or inorganic acid solution.

In yet another embodiment of the present invention, there is provided a method, wherein the non-polar solvent used in the extraction is selected from hexane, heptane, chloroform and acetone.

In still another embodiment of the present invention, there is provided a method, wherein the polar solvent is selected from acidic ethanol, methanol, dimethyl sulfoxide (DMSO) and dimethylformamide (DMF).

In an embodiment of the present invention, there is provided a method, wherein the polar solvent is acidic ethanol.

In another embodiment of the present invention, there is provided a method, wherein the ratio of biomass to acidic ethanol is in the range of 1:5- 1:60.

In yet another embodiment of the present invention, there is provided a method, wherein the moisture adsorbent is selected from activated alumina, sodium sulphate, silica gel or calcium chloride.

In still another embodiment of the present invention, there is provided a method, wherein the extraction mixture comprises ethanol and hydrochloric acid.

In an embodiment of the present invention, there is provided a method, wherein the obtained FAAEs are rich in omega-3 fatty acids selected from palmitic acid, oleic acid, Docosahexaenoic acid (DHA), Docosapentaenoic acid (DPA) or Eicosapentaenoic acid (EPA).

In another embodiment of the present invention, there is provided a method, wherein the FAEEs are obtained within 4 hours.

These and other features, aspects, and advantages of the present subject matter will be better understood with reference to the following description. This summary is provided to introduce a selection of concepts in a simplified form.

### BRIEF DESCRIPTION OF DRAWINGS

These and other features, aspects, and advantages of the present invention will become better understood when the following detailed description is read with reference to the accompanying drawings in which like characters represent like parts throughout the drawings wherein:
**Figure 1** illustrates comparison of fatty acid composition processed by Folch method and direct transesterification.
**Figure 2** illustrates the effect of biomass to solvent ratio on extraction yields from dry biomass.

### DETAILED DESCRIPTION OF THE INVENTION

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

### Definition:

For the purposes of this invention, the following terms will have the meaning as specified therein:
The articles "a", "an" and "the" are used to refer to one or to more than one (i.e., to at least one) of the grammatical object of the article.
The terms "comprise" and "comprising" are used in the inclusive, open sense, meaning that additional elements may be included. It is not intended to be construed as "consists of only". Throughout this specification, unless the context requires otherwise the word "comprise", and variations such as "comprises" and "comprising", will be understood to imply the inclusion of a stated element or step or group of element or steps but not the exclusion of any other element or step or group of element or steps.
The term "including" is used to mean "including but not limited to" "including" and "including but not limited to" are used interchangeably.

The present invention relates to a rapid method for downstream processing for *in-situ* synthesis of alkyl esters of fatty acids and their efficient extraction from wet biomass slurry. Particularly, the present invention relates to a method to eliminate the necessity of dewatering of biomass slurry by biomass harvesting and drying before FAAEs synthesis and extraction from oleaginous biomass. The method disclosed in the present invention increases the transesterification efficiency (>95%) of FAAEs production from wet biomass without adding large volume of solvents such as ethanol or methanol to overcome the negative effect of water presence in the reaction. The method results in more than 95% FAAE transesterification efficiency from treated oleaginous biomass slurry via two step *in-situ* transesterification reaction. Obtained FAAEs from wet oleaginous biomass slurry can be directly used as biodiesel or in mix with diesel fuel. These alkyl esters of fatty acids also have wider applications in pharmaceutical as well as in nutraceutical industry due to presence of higher amount of omega-3 fatty acids like Docosahexaenoic acid (DHA), Docosapentaenoic acid (DPA), Eicosapentaenoic acid (EPA). Accordingly, the present invention provides a means of downstream processing of wet oleaginous biomass slurry for fatty acid alkyl ester synthesis and their extraction while eliminating the necessity of biomass harvesting, drying and large requirement of chemical solvents.

Thus, in accordance with the present invention, there is provided a rapid downstream processing method for synthesis and extraction of fatty acid alkyl ester from an oleaginous microbe, the method comprising:
(i) subjecting wet biomass slurry to osmotic and acidic treatment;
(ii) mixing the treated biomass slurry of step (i) having moisture content in the range of 80% - 90% with an extraction mixture;
(iii) adding non-polar solvent to the mixture of step (ii) to obtain an extract;
(iv) subjecting the extract to evaporation and treating with a polar solvent; and
(v) adding moisture adsorbent to the extract to obtain fatty acid alkyl ester (FAAE) rich in omega-3 fatty acids with the non-polar solvent,
wherein the addition of moisture adsorbent at step (v) increases the FAEE transesterification efficiency up to 95%.

In an embodiment of the present invention, there is provided a method wherein the fatty acid alkyl ester is selected from methyl ester, ethyl ester and propyl ester. The method of the present invention can be used to obtain fatty acid methyl ester, fatty acid ethyl ester and fatty acid propyl esters etc. depending upon the addition of alkyl group donating solvent in the reaction.

In another embodiment of the present invention, there is provided a method wherein the oleaginous microbe is a heterotrophic microalga belonging to the order Thraustochytrids. In the present invention, the Thraustochytrids were cultivated in different volumes in culture flasks including but not limited to 50 mL, 100 mL 250 mL flasks or in fermenters including but not limited to 2 L fermenters, 10 L fermenters, 60L fermenters, 100 L fermenters or 500 L fermenters.

In a preferred embodiment of the present invention, the oleaginous microbe is a microalga. Particularly, the microalgae is *Schizochytrium sp.* DBT-IOC1 (MTCC 5890). The *Schizochytrium* MTCC 5980 used in the present invention was isolated from Zuari- Mandovi mangroves, in Goa, India (S15°29'57.39", E73°52'6.13") near the Arabian Sea.

In yet another embodiment of the present invention, there is provided a method wherein the wet biomass slurry is directly harvested from the fermenter.

In still another embodiment of the present invention, there is provided a method wherein osmotic treatment in step (i) comprises treating the wet biomass slurry with single distilled water or double distilled water or triple distilled water or water having very low TDS.

In an embodiment of the present invention, there is provided a method wherein acidic treatment in step (i) comprises treating the wet biomass slurry with an organic or inorganic acid solution. In the present method, the wet biomass slurry is subjected to both osmotic treatment and acid treatment. The osmotic and acidic treatment of slurry results in a synergistic effect. Further, the osmotic and acidic washing or treatment results into reduction in ash content of biomass slurry.

In another embodiment of the present invention, there is provided a method wherein the non-polar solvent used in the extraction is selected from hexane, heptane, chloroform, and acetone. In a preferred embodiment, the non-polar solvent used in the extraction is hexane.

In yet another embodiment of the present invention, there is provided a method wherein the polar solvent is selected from acidic ethanol, methanol, dimethyl sulfoxide (DMSO) and dimethylformamide (DMF). In a preferred embodiment, the polar solvent is acidic ethanol.

In still another embodiment of the present invention, there is provided a method wherein the ratio of biomass to acidic ethanol is in the range of 1:5- 1:60. In a preferred embodiment of the present invention, the ratio of biomass to acidic ethanol is 1: 10.

In an embodiment of the present invention, there is provided a method wherein the moisture adsorbent is selected from activated alumina, sodium sulphate, silica gel or calcium chloride. In a preferred embodiment, the moisture adsorbent is activated alumina or sodium sulphate.

In another embodiment of the present invention, there is provided a method wherein the extraction mixture comprises ethanol and hydrochloric acid.

In yet another embodiment of the present invention, there is provided a method wherein the obtained FAAEs are rich in omega-3 fatty acids selected from palmitic acid, oleic acid, Docosahexaenoic acid (DHA), Docosapentaenoic acid (DPA) or Eicosapentaenoic acid (EPA). Further, the omega-3 fatty acid content in the range of 15% - 50 % or more of total fatty acids. The present method also reduces the chances of oxidation or epoxidation of omega-3 fatty acids.

In still another embodiment of the present invention, there is provided a method wherein the FAAEs are obtained within 4hours from the time of broth harvesting.

In an embodiment of the present invention, there is provided a method which further comprises washing FAAEs with 2% KHCO₃ and passing through anhydrous Na₂SO₄ for removal of moisture.

The present invention discloses a rapid and less energy intensive downstream processing method for obtaining FAAEs directly from wet oleaginous biomass slurry without harvesting and drying of wet biomass, which helps in reducing the downstream processing time and chances of oxidation or epoxidation of omega-3 fatty acids, since omega-3 fatty acids are very prone to oxidation/epoxidation due to multiple double bonds. Further, the present method results in more than 95% of FAAE (biodiesel) from wet oleaginous biomass. The present method eliminates the need of cost and energy intensive steps of dewatering of biomass including harvesting and drying. The slurry or fermentation broth from fermenters can directly processed with this method. Moreover, since this method can be also deployed as continuous method of downstream processing of wet oleaginous biomass therefore, it can be integrated with continuous fermentation system, where fermentation broth after exiting fermenters is continuously processed to obtain FAAEs. Therefore, the method disclosed in the present invention provides a downstream processing method for *in-situ* synthesis of alkyl ester of fatty acids and their extraction directly from wet oleaginous biomass slurry without involving any biomass harvesting methods. Also, the method eliminates the need of biomass drying before lipid/oil extraction and transesterification without compromising fatty acid alkyl ester (FAAE) extraction yield from wet oleaginous biomass slurry.

### EXAMPLES

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods, the exemplary methods, devices, and materials are described herein. It is to be understood that this disclosure is not limited to methods, and experimental conditions described, as such methods and conditions may vary.

### Example-1: Cultivation of Oleaginous (Thraustochytrid) biomass

A continuous Thraustochytrid fermentation system was set up in 100L fermenter having working volume of 60L. Fermenter, having 60L acetate rich minimal growth media, was inoculated with 48 hours old 3L culture of *Schizochytrium sp.* DBTIOC-1 (MTCC 5890). The microalgal strain *Schizochytrium* MTCC 5980 was isolated from Zuari- Mandovi mangroves, in Goa, India (S15°29'57.39", E73°52'6.13") near the Arabian Sea.

Fermenter was continuously aerated with air and oxygen along with agitation to maintain required dissolved oxygen level. Fermenter was continuously fed with media while broth was continuously taken out through level control. Broth was collected in 200L tank. This broth was then centrifuged at 4000 rpm for 15 minutes to remove depleted media components and water. Pellets were washed with distilled water twice before drying. This biomass slurry was still containing more than 75% of moisture therefore, the biomass slurry was dried at 80°C in large trays in hot air oven for 4-5 days. Dried biomass flakes were then powdered with mixer grinder for lipid extraction and fatty acid ethyl ester synthesis. Some part of fermentation broth was stored at -20°C to study *in-situ* transesterification of lipids into fatty acid ethyl esters later from wet algal biomass slurry.

### Example-2 Comparison of Folch method with Direct transesterification or in-situ ethyl ester synthesis method

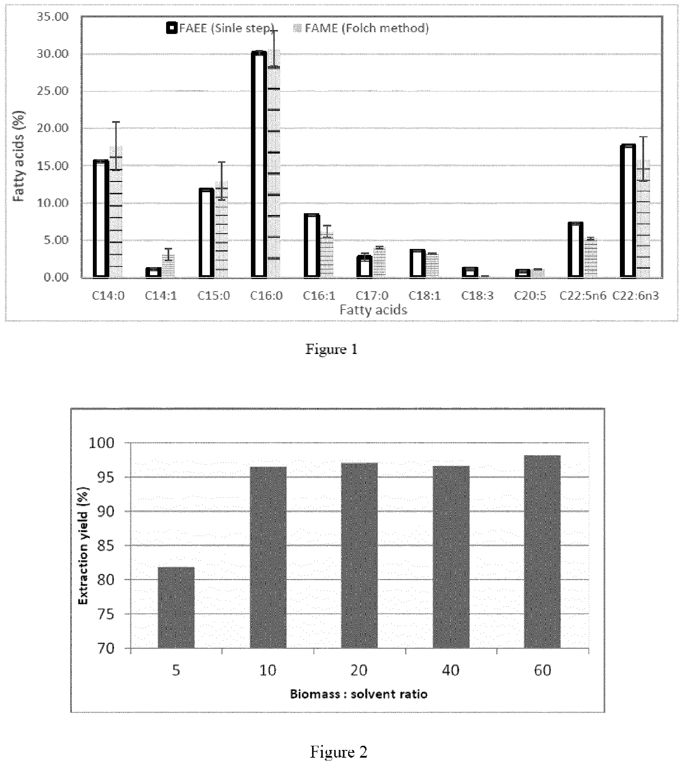

Total lipid content of biomass was estimated by the modified Folch method using chloroform and ethanol (2:1) solvent mixture in the ratio of 1:60 (dry biomass: solvent mixture). Dried powder of biomass (20g) was added in 3L round bottom flasks having 1.2 L solvent mixture and agitated at 100 rpm at 70°C for 1 hour. Whole mixture was centrifuged at 4500 rpm for 15 minutes for solid liquid separation. Supernatant was transferred in separating funnel followed by addition of hexane for phase separation. Upper layer containing lipid was carefully collected followed by solvent evaporation in Rotary evaporator. All the steps were repeated thrice for complete extraction of lipid from biomass. Lipid was measured gravimetrically followed by FAEE synthesis and analysis as per protocol given by Gupta *et. al.* in 2016. Total FAEE content was calculated gravimetrically as well as using GC-FID data and these values were taken as a reference to calculate FAEE extraction yield of different samples (Fig.1).

In Direct transesterification method, 20g of dry biomass powder was mixed with 1.2L of extraction mixture having 1.18L ethanol and 20ml of HCl (biomass to solvent ratio 1:60) and agitated at 100 rpm at 90°C for 1 hour. Whole mixture was transferred in separating funnel followed by addition of hexane for phase separation. Upper layer containing FAEE was carefully harvested followed by hexane evaporation in Rotary evaporator. FAEE was calculated gravimetrically as well as with GC-FID to quantify FAEE extraction yield and FAEE transesterification efficiency. FAEE content (5.25 g) was found to be comparable with that of Folch method (5.4g) suggesting complete extraction of FAEE from biomass in direct transesterification with FAEE transesterification efficiency of more than 97% (Table 1). This reflects that almost all of lipid, present in biomass, is converted into ethyl esters of fatty acid without any significant change in fatty acid composition (Figure 1). Biomass to ethanol ratio was further optimized to reduce the amount of ethanol needed for the reaction. 20g of biomass was added in 1.18L or 0.78 L or 0.38 L or 0.18 L or 0.08L of ethanol while amount (20ml) of acid catalysts i.e., HCl was same in all the reactions. FAEE extraction yield was almost equal till biomass to solvent ratio of 1:10, but there here was significant reduction in FAEE extraction yield when biomass to solvent ratio was reduced to 1:5 suggesting almost complete FAEE extraction when biomass to solvent ratio of 1:10 was applied for the reaction (Figure 2).

**Table: 1 Comparison of Folch method and Direct transesterification method**

| **Parameters** | **Folch method (Reference)** | **Direct transesterification** |
|---|---|---|
| FAEE Content (g) from 20g dry biomass | 5.4 | 5.3 |
| FAEE extraction yield (%) | 100 | 98.14 |
| FAEE Extraction efficiency (%) | 100 | 97.5 |
| C16:0 (%) | 30.68 | 30.08 |
| C22:6n3 (%) | 15.86 | 17.64 |

### Example-3: Production of FAEE from present method (Direct transesterification or in-situ ethyl ester synthesis of FAEE) from dry and wet biomass

100g to 110g of wet biomass paste or slurry, having 80%-90% moisture content, was taken for *in-situ* synthesis of FAEE and their FAEE extraction yields were compared with dry biomass as per direct transesterification method given in Example 2. FAEE extraction yield from wet biomass was found to be almost 30% less than dry biomass suggesting incomplete extraction (Table 2). This implies the fact that cell wall is acting as barrier in solvent accessibility of lipid hence needs to be disrupted for better extraction. To overcome the issue of incomplete extraction, wet biomass pastes or slurry or fermentation broth was directly washed with 0.3N HCl (for acidic treatment) or low TDS water (for osmotic shock) or both. There was not much improvement in FAEE extraction yield when slurry was washed with acid or water alone however when both were combined there was significant improvement in FAEE extraction yield (Table 3). Subsequently this step was added before proceeding for FAEE extraction from wet biomass slurry. Although FAEE extraction yield was equal to that from dry biomass, but FAEE transesterification efficiency was still very low from 50% to 60% of total lipid suggesting incomplete transesterification reaction and making it unsuitable for biodiesel application (Table 4).

**Table 2: Comparison of extraction yields from dry biomass and wet algal biomass slurry**

| **Parameters** | **Dry biomass** | **Wet algal biomass slurry** |
|---|---|---|
| FAEE Content (g) from 20 g dry biomass | 5.21 | 3.69 |
| FAEE content (%) | 26.05 | 18.45 |
| FAEE extraction yield (%) in comparison to | 96.5 | 68.33 |

**Table 3: Effect of different washing methods on extraction yields from wet algal biomass slurry**

| | **Wet algal biomass slurry** | | | |
|---|---|---|---|---|
| **Parameters** | **With no treatment** | **Acid treatment** | **Osmotic treatment** | **Osmotic + acidic** |
| FAEE Content (g) from 20 g dry biomass | 3.69 | 4.15 | 4.1 | 5.16 |
| FAEE content (%) | 18.45 | 20.75 | 20.5 | 25.82 |
| FAEE extraction yield (%) in comparison to Folch method | 68.33 | 76.85 | 75.92 | 95.55 |

### Example-4: Increasing the transesterification efficiency for in-situ ethyl ester synthesis of fatty acids from wet Thraustochytrid biomass slurry

Low transesterification efficiency from wet biomass slurry or broth is reported to be due to the presence of water in the reaction. Therefore, the method for direct transesterification was further modified to negate the effect of water on FAEE transesterification efficiency. Particularly, moisture adsorbents such as activated alumina or sodium sulphate were added in the reaction while a second transesterification step was added in the protocol. Sequence of moisture adsorbents addition in multistep transesterification reaction was reported to have effect on FAEE transesterification efficiency. Hexane fraction was collected from direct transesterification method as given in Example 3 followed by solvent evaporation under rotary evaporator. FAEE extract was subjected to a second transesterification reaction with acidic ethanol and FAEE was subsequently extracted with hexane. FAEE transesterification efficiency increased from 52.5% to 72.9% (reaction condition 1), however this is still short of required efficiency of over 95% for biodiesel application (Table 4). In another experiment different amount of alumina or sodium sulphate i.e., 5g or 10g or 20g was added in reaction mixture (180mL ethanol plus 20mL HCl) as given in Example 3 followed by hexane extraction. Subsequently, hexane was evaporated and FAEE was subjected to a second transesterification reaction. Addition of moisture adsorbent resulted into increase in FAEE transesterification efficiency from 52.5% to 87.8% (reaction condition 2). When moisture adsorbent was added in second transesterification reaction instead of first, FAEE transesterification efficiency significantly increased from 55% to 96.5% (reaction condition 3).

**Table.4 Effect of different transesterification reaction conditions on FAEE transesterification efficiency**

| | **Wet algal biomass slurry** | | | |
|---|---|---|---|---|
| **Parameters** | **Control** | **Reaction condition-** | **Reaction condition-2** | **Reaction condition- 3** |
| FAEE Content (g) from 20g dry biomass | 5.16 | 5.25 | 5.20 | 5.16 |
| FAEE content (%) | 25.82 | 26.25 | 26.00 | 25.82 |
| FAEE extraction yield (%) in comparison to Folch method | 95.55 | 97.22 | 96.29 | 95.55 |
| FAEE Extraction efficiency (%) | 52.15 | 72.9 | 87.8 | 96.5 |

### Example-5: Analysing FAEE by GC-FID

FAEEs were washed with 2% KHCO₃ and passed through anhydrous Na₂SO₄ for removal of any moisture if present. FAEEs were concentrated under nitrogen or rotary evaporator and analyzed with GC-FID system (Perkin Elmer Clarus 680, US), equipped with fast-GC capillary column (Omegawax 100 column of dimension 15 m x 0.1 mm, 0.1 µm thickness). Sample of FAEEs (1µl) was injected while maintaining injector temperature at 250°C. Oven temperature was ramped up at the rate of 40°C/sec from 140°C to final 280°C and hold for 2 minutes. Detector was set at 260°C. Hydrogen was used as carrier gas with velocity rate 50 cm sec⁻¹. Fatty acid peaks were identified and quantified with Total chrome chromatography software (Perkin Elmer, US).

## Claims

1. A rapid downstream processing method for synthesis and extraction of fatty acid alkyl ester from an oleaginous microbe, the method comprising:
(i) subjecting wet biomass slurry to osmotic and acidic treatment;
(ii) mixing the treated biomass slurry of step (i) having moisture content in the range of 80% - 90% with an extraction mixture;
(iii) adding non-polar solvent to the mixture of step (ii) to obtain an extract;
(iv) subjecting the extract to evaporation and treating with a polar solvent; and
(v) adding moisture adsorbent to the extract to obtain fatty acid alkyl ester (FAAE) rich in omega-3 fatty acids with the non-polar solvent,
wherein the addition of moisture adsorbent at step (v) increases the FAEE transesterification efficiency up to 95%.

2. The method as claimed in claim 1, wherein the obtained fatty acid alkyl ester is selected from methyl ester, ethyl ester and propyl ester.

3. The method as claimed in claim 1, wherein in step (ii) the treated biomass slurry is mixed with extraction mixture and is agitated at 100 rpm at a temperature of 90°C for a duration 1 hour.

4. The method as claimed in claim 1, wherein the oleaginous microbe is a microalga.

5. The method as claimed in claim 1, wherein the wet biomass slurry is directly harvested from the reactor.

6. The method as claimed in claim 1, wherein the osmotic treatment in step (i) comprises treating the wet biomass slurry with single distilled water or double distilled water or triple distilled water or water having very low TDS.

7. The method as claimed in claim 1, wherein acidic treatment in step (i) comprises treating the wet biomass slurry with an organic or inorganic acid solution.

8. The method as claimed in claim 1, wherein the non-polar solvent used in the extraction is selected from hexane, heptane, chloroform, and acetone.

9. The method as claimed in claim 1, wherein the polar solvent is selected from acidic ethanol, methanol, dimethyl sulfoxide (DMSO) and dimethylformamide (DMF).

10. The method as claimed in claim 9, wherein the polar solvent is acidic ethanol.

11. The method as claimed in claims 1-10, wherein the ratio of biomass to acidic ethanol is in the range of 1:5- 1:60.

12. The method as claimed in claim 1, wherein the moisture adsorbent is selected from activated alumina, sodium sulphate, silica gel or calcium chloride.

13. The method as claimed in claim 1, wherein the extraction mixture comprises ethanol and hydrochloric acid.

14. The method as claimed in claim 1, wherein the obtained FAAEs are rich in omega-3 fatty acids selected from palmitic acid, oleic acid, Docosahexaenoic acid (DHA), Docosapentaenoic acid (DPA) or Eicosapentaenoic acid (EPA).

15. The method as claimed in claim 1, wherein the FAEEs are obtained within 4 hours.
